# EUROPÄISCHE PATENTSCHRIFT

(11) **EP 3 777 739 B1**
(45) Veröffentlichungstag und Bekanntmachung des Hinweises auf die Patenterteilung: **20.04.2022**
(21) Anmeldenummer: 19191757.4
(22) Anmeldetag: 14.08.2019
(51) Int. Cl.: A61B 18/12, A61B 18/14, A61B 18/00, B26D 1/00, B26F 3/00, B29C 59/00, B29C 65/00

(54) **SCHNEIDELEKTRODE, CHIRURGISCHES INSTRUMENT UND VERFAHREN ZUR HERSTELLUNG DER SCHNEIDELEKTRODE**
CUTTING ELECTRODE, SURGICAL INSTRUMENT AND METHOD OF MANUFACTURING THE CUTTING ELECTRODE
ÉLECTRODE DE DÉCOUPAGE, INSTRUMENT CHIRURGICAL ET PROCÉDÉ DE FABRICATION D'UNE ÉLECTRODE DE DÉCOUPAGE

(43) Veröffentlichungstag der Anmeldung: 17.02.2021
(73) Patentinhaber: Erbe Elektromedizin GmbH, 72072 Tübingen (DE)
(72) Erfinder: KOERNER, Johannes, 88239 Wangen im Allgäu (DE)
(74) Vertreter: Rüger Abel Patentanwälte PartGmbB

(56) Entgegenhaltungen:
- EP-A1- 2 617 378
- EP-A1- 2 959 854
- EP-A2- 1 958 583
- WO-A1-2011/083027

## Beschreibung

Die Erfindung betrifft ein Verfahren zur Herstellung einer Schneidelektrode für ein chirurgisches Instrument, insbesondere ein Fusions- und Dissektionsinstrument sowie die mit dem Verfahren hergestellte Schneidelektrode und ein chirurgisches Instrument, das eine entsprechende Schneidelektrode aufweist.

Aus der US 8 740 901 B2 und aus der EP 2 959 854 A1 ist jeweils ein chirurgisches Instrument zur Gefäßversiegelung und Durchtrennung, d.h. zur Gewebefusion und Dissektion bekannt, bei dem sowohl die Gewebeversiegelung als auch der Gewebeschnitt elektrisch durchgeführt werden. Zur Gefäßversiegelung dienen an Backen zweier Branchen ausgebildete Versiegelungselektroden, die einen Strom durch ein zwischen den Backen gefasstes Gewebe leiten, das sich dadurch erhitzt und koaguliert. Auf diese Weise sind von dem Instrument gefasste Gefäße verschließbar. Eine in einer der Branchen untergebrachte Schneidelektrode dient dabei zur Gefäßdurchtrennung. Die Schneidelektrode kann dabei, je nach Ausführungsform, verschieden tief in ein Isolationsmaterial eingebettet sein, sodass von der Schneidelektrode lediglich ihre Schmalseite und Teile ihrer Flachseiten freiliegen.

Aus der EP 0 852 480 B1 ist ein skalpellartiges elektrochirurgisches Instrument bekannt, dessen flache spatelförmige Elektrode an ihren beiden Flachseiten mit einer dicken isolierenden Beschichtung versehen ist. Diese Beschichtung überdeckt auch die Schmalseiten, ist dort jedoch relativ dünn. Demgemäß soll ein von dem Instrument ausgehender HF-Strom vorwiegend durch die Kanten des Instruments fließen.

Ein solches Instrument ist auch aus der EP 3 132 765 A1 bekannt. Es weist in seiner oberen, mit einer Schneidelektrode versehenen Branche einen Isolatorkörper auf, der mittig einen schmalen, auf ein Widerlager der gegenüberliegenden Branche hin gerichteten Wandabschnitt aufweist. In die Schmalseite dieses Wandabschnitts ist die Schneidelektrode eingelassen.

Der Aufbau eines Isolatorkörpers mit Schneidelektrode kann prinzipiell auch der US 2016/0249975 A1 entnommen werden. Die Schneidelektrode wird durch schmale streifenförmige Metallabschnitte gebildet, die unterschiedliche ohmsche Widerstände aufweisen.

Weiterer Stand der Technik wird durch die US 8, 394, 094 B2 gebildet. Diese schlägt vor, eine Schneidelektrode zwischen zwei Isolatorelementen zu halten, die die Flachseiten der Schneidelektrode weitgehend bedecken und die Schmalseite der Schneidelektrode freilassen.

Der Trend bei chirurgischen Instrumenten geht zu filigranen dabei aber hoch leistungsfähigen und zuverlässig arbeitenden Instrumenten. Solche Instrumente müssen in kurzer Zeit einen möglichst präzisen Schnitt erzeugen. Dies möglichst mit geringem Stromaufwand, um zugleich eine Gewebekoagulation durchführen zu können. Insbesondere in Fällen, bei denen die Koagulationselektroden und die Schneidelektrode gleichzeitig aus ein und demselben Generator mit beschränkter Leistung gespeist werden, kann es von einiger Bedeutung sein, einen stromsparenden Schnitt zu erreichen.

Es hat sich gezeigt, dass es dabei auf die Geometrie der Schneidelektrode und ihre Isolierung maßgeblich ankommt. Entsprechende Schneidelektroden sind aber schwierig zu fertigen.

Davon ausgehend, ist es Aufgabe der Erfindung, ein Verfahren zur Herstellung einer Schneidelektrode anzugeben.

Diese Aufgabe wird mit dem Verfahren nach Anspruch 1 gelöst:
Das erfindungsgemäße Verfahren beruht auf der Bereitstellung eines flachen Materialzuschnitts aus einem elektrisch leitfähigen Material, vorzugsweise eines Blechzuschnitts, der sowohl einen die spätere Elektrode bildenden Abschnitt als auch einen lediglich der Handhabung dienenden Abschnitt aufweist. Dieser Materialzuschnitt wird mit einer Sollbruchstelle versehen, die ihn in den ersten und zweiten Abschnitt unterteilt. Der erste Abschnitt dient lediglich der Handhabung während der zweite Abschnitt die spätere Elektrode bildet. Der Materialzuschnitt ist beispielsweise ein dünnes Metallblech, dessen Dicke, je nach Anwendungsfall, von Bruchteilen eines 1/10 Millimeters bis zu mehreren 1/10 Millimetern reichen kann. Bei einem bevorzugten Ausführungsbeispiel beträgt die Dicke 0,1 mm. Das Metall kann Stahl sein, wobei der Stahl vorzugsweise nicht duktil, insbesondere nicht plastisch verformbar ist ist.

Die Sollbruchstelle wird vorzugsweise durch eine linienhafte Zone gebildet, in der die Dicke des Blechzuschnitts reduziert ist. Die Dickenreduktion kann durch eine gerade oder bogenförmige Rille gebildet werden, die sich über eine Flachseite des Materialzuschnitts vorzugsweise von einer Kante bis zu einer anderen Kante desselben erstreckt. Alternativ können auch zwei Rillen oder Nuten vorgesehen sein, die an den beiden gegenüberliegenden Flachseiten des Materialzuschnitts angeordnet sind und gleiche Position und gleichen Verlauf aufweisen. Mit anderen Worten, sie sind parallel zueinander orientiert und legen eine Fläche fest, die senkrecht auf in Flachseiten des Materialzuschnitts steht.

Die Sollbruchstelle kann durch Schleifen, Fräsen, Ätzen, Prägen, Rollieren oder ähnliche Verfahren erzeugt werden, die geeignet sind, die Dicke des Materialzuschnitts lokal zu verringern. Es ist darüber hinaus auch möglich, die Sollbruchstelle durch eine Reihe von Perforationen oder Schlitzen oder durch eine lokale Materialveränderung, zum Beispiel lokale Materialversprödung zu bilden. Diese kann thermisch verursacht werden, zum Beispiel durch lokales Laserhärten.

Im nächsten Schritt wird der mit der Sollbruchstelle versehene Materialzuschnitt in die Gravur einer Gießform eingelegt, die den ersten Abschnitt anliegend umfasst, sodass der zweite Abschnitt in eine Kaverne bzw. einen Hohlraum der Gießform ragt. Dabei ist die Gießform vorzugsweise so ausgebildet, dass die Sollbruchstelle genau am Rand des Hohlraums positioniert ist. Im nächsten Schritt wird Kunststoff in die Gießform eingefüllt, sodass dieser den zweiten Abschnitt umfließt und gegebenenfalls auch in dem zweiten Abschnitt vorgesehene Perforationen durchsetzt. Es ergibt sich somit ein Formschluss zwischen dem Kunststoffkörper und dem zweiten Abschnitt. Das bevorzugte Gießverfahren ist Spritzgießen.

Nach dem Öffnen der Gießform und dem Entnehmen des Kunststoffkörpers mit dem darin gefassten Materialzuschnitt wird der erste Abschnitt des Materialzuschnitts, beispielsweise von Hand oder mittels einer Zange oder einem anderen geeigneten Werkzeugs an der Sollbruchstelle von dem zweiten Abschnitt abgebrochen. Es bildet sich damit entlang des Rands des Kunststoffkörpers eine Bruchkante, an der der zweite Abschnitt des Materialzuschnitts als nun fertige Schneidkante freiliegt.

Die Schneidkante ist somit bruchrau und sie bedarf keiner Nacharbeitung. Beschädigungen oder Belastungen des Kunststoffkörpers durch nachfolgende Bearbeitungsschritte werden minimiert. Das Metallblech der Schneidelektrode ist in dem Kunststoffkörper vorzugsweise symmetrisch angeordnet.

Das Verfahren ermöglicht die Erzeugung einer elektrisch leitfähigen fast linienartigen, vorzugsweise metallischen, Schneidkante unmittelbar an dem ein- oder mehrteilig ausbildbaren Kunststoffkörper, ohne dass Teile der Flachseite der Schneidelektrode freiliegen müssten. Es sind für das Abtrennen des ersten Abschnitts keine Flächenbereiche für Greifer, Zangen oder ähnliches frei zu halten. Die Schneidkante kann bündig mit dem Kunststoffkörper abschließen, ganz leicht über diesen überstehen oder auch etwas unterhalb der angrenzenden Flächen des Kunststoffkörpers liegen. Die Fertigung ist somit einfach und prozesssicher in hoher Qualität möglich und gestattet wegen der Restriktion des Stroms auf eine schmale Bruchkante der Schneidelektrode die Erzielung höchster Schneidqualitäten. Die Schneidelektrode kann dabei aus Metall oder einer elektrisch leitfähigen Keramik bestehen. Das erfindungsgemäße Verfahren ermöglicht die besonders präzise Herstellung der Schneidelektrode, bei der die Elektrode mittig in den Kunststoffkörper eingebettet ist, so dass die Kunststoffwände zu beiden Seiten der Elektrode gleich dick sind.

Wenn der erste Abschnitt des Materialzuschnitts wenigstens so groß ist, wie der zweite Abschnitt, ist die Handhabung während der Fertigung und das spätere Abtrennen des ersten Abschnitts vom übrigen mit dem Kunststoffkörper umspritzten zweiten Abschnitt sehr einfach auch von Hand zu erledigen.

Wenn der erste Abschnitt mittels mindestens eines, vorzugsweise mindestens zweier Stifte in der Gießform lagerichtig positioniert wird, ergibt sich eine besonders sichere Positionierung des Materialzuschnitts in der Gießform.

Die Sollbruchstelle ist vorzugsweise als ein schmaler streifenförmiger, d.h. linienhafter Bereich verminderter Materialstärke ausgebildet. Dieser kann durch ein oder beidseitiges Schleifen, Rollieren oder Ätzen des Materialzuschnitts oder anderweitig ausgebildet werden. Der Bereich verminderter Materialstärke kann eine Nut sein. Diese Nut kann einen runden, rechteckigen, trapezförmigen, dreieckigen oder bogenförmigen Querschnitt aufweisen. Diese Nut ist unabhängig von ihrer Form vorzugsweise wenigstens ein Drittel, weiter vorzugsweise wenigstens halb so tief wie die Dicke des Materialzuschnitts. Sowohl die Nuttiefe als auch die Dicke des Materialzuschnitts sind senkrecht zu einer Flachseite des Materialzuschnitts zu messen. Sind beidseitig Nuten in den Materialzuschnitt eingebracht, beträgt deren Tiefe vorzugsweise 1/6 bis 1/4 der Dicke des Materialzuschnitts.

Die mit dem vorbeschriebenen erfindungsgemäßen Verfahren erzeugte Schneidelektrode weist einen Kunststoffkörper auf, in den eine Elektrode eingebettet ist, die aus dem zweiten Abschnitt des Materialzuschnitts hervorgegangen ist. Sie weist eine freiliegende Bruchkante auf, die die elektrische Schneidkante bildet.

Vorzugsweise besteht der Kunststoffkörper aus einem flexiblen Kunststoff, insbesondere einem elastischen Kunststoff, wie beispielsweise Silikon, oder aus einem silikonartigen Kunststoff, einem Elastomer oder dergleichen.

Die Elektrode weist vorzugsweise eine einheitliche konstante Dicke auf, wobei sie in unmittelbarer Nachbarschaft der Bruchkante eine reduzierte Dicke aufweisen kann, sodass eine sich an die Bruchkante anschließende Verjüngungszone ausgebildet ist. Diese Verjüngungszone trägt zur Stromkonzentration an der Schneidkante bei, sodass ein schneller sauberer Schnitt erzielbar ist.

Der Kunststoffkörper ist vorzugsweise mindestens an die Verjüngungszone heranreichend und weiter vorzugsweise in die Verjüngungszone hineinreichend oder diese übergreifend ausgebildet, sodass die freiliegende Bruchfläche der Elektrode vorzugsweise nicht größer ist als die Querschnittsfläche der Schneidelektrode. Dies bedeutet, dass die Flanken der Elektrode vorzugsweise bis zur Bruchkante von Kunststoff bedeckt sind.

Zu beiden Seiten der Bruchkante weist der Kunststoffkörper Stirnflächen auf, deren Breite vorzugsweise kleiner ist als das doppelte der Dicke (Breite) der Elektrode. Dies vermeidet eine Gewebequetschung und mechanische Durchtrennung und sichert, dass der gewünschte Schnitt ganz oder ganz vorwiegend elektrisch nicht aber mechanisch ausgeführt wird.

Weitere Einzelheiten vorteilhafter Ausführungsformen der Erfindung ergeben sich aus der Zeichnung, der Beschreibung oder Ansprüchen. Es zeigen:
Figur 1 ein chirurgisches Instrument, in beispielhafter Ausführung und perspektivischer vereinfachter Darstellung,
Figur 2 ein von dem Instrument nach Figur 1 getragenes Werkzeug, in vereinfachter Vertikalschnittdarstellung,
Figur 3 eine Schneidelektrode des Werkzeugs nach Figur 2, in vereinfachter Perspektivdarstellung,
Figur 4 einen Materialzuschnitt zur Herstellung einer Schneidelektrode, in Seitenansicht,
Figur 5 eine Gießform mit einem Materialzuschnitt, in Explosionsdarstellung geschnitten senkrecht zur Trennebene der Gießform,
Figur 6 einen Materialzuschnitt, in ausschnittsweiser Schnittdarstellung, im Bereich seiner Sollbruchstelle,
Figur 7 eine alternative Ausführungsform eines Materialzuschnitts, in ausschnittsweiser Schnittdarstellung, im Bereich seiner Sollbruchstelle,
Figur 8 einen mit einem Kunststoffkörper versehenen Materialzuschnitt zur Herstellung einer Schneidelektrode,
Figur 9 eine Schneidelektrode, in einer vertikal geschnittenen ausschnittsweisen Darstellung, in einer ersten Ausführungsform, und
Figur 10 eine Schneidelektrode, in einer Darstellung gemäß Figur 9, jedoch in abgewandelter Ausführungsform.

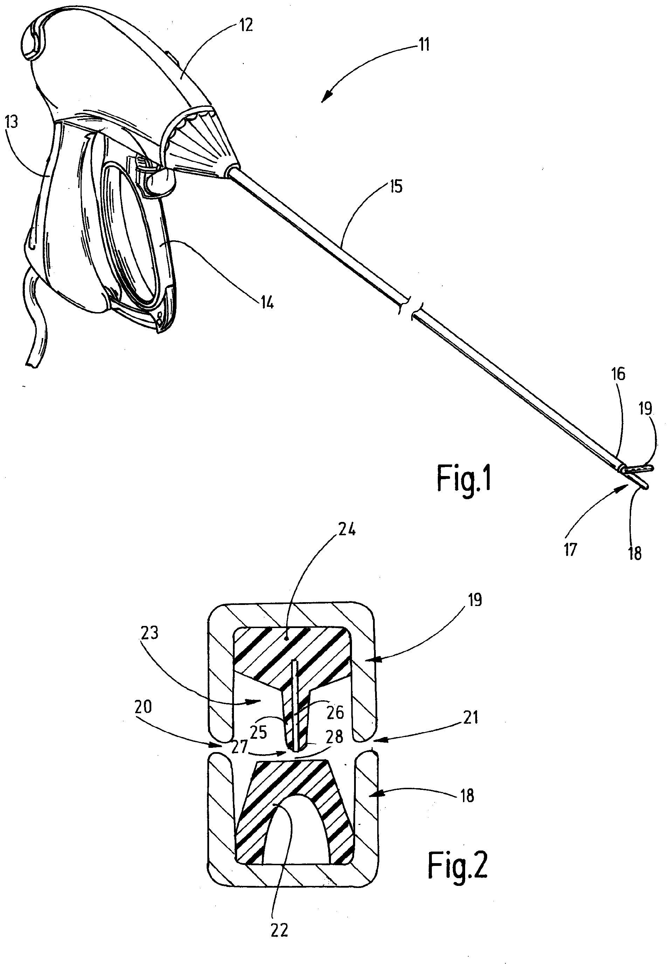

Figur 1 veranschaulicht ein chirurgisches Instrument 11, das insbesondere zur laparoskopischen Behandlung eines Patienten vorgesehen ist. Das Instrument 11 weist ein Gehäuse 12 mit einem daran vorgesehenen Handgriff 13 und einem Betätigungshebel 14. Von dem Gehäuse 12 erstreckt sich ein Schaft 15 weg, an dessen distalen Ende 16 ein Werkzeug 17 gehalten ist. Dieses weist eine erste Branche 18 und eine zweite Branche 19 auf, von denen wenigstens eine durch Betätigung des Handhebels 14 auf die andere zu und von dieser weg bewegbar ist, um Gewebe zwischen einander fassen zu können.

Die Branchen 18, 19 bilden das zangenartige Werkzeug 17, das zur Gewebeversiegelung und/oder zur Gewebetrennung vorgesehen ist. Dazu können die beiden Branchen 18, 19, wie beispielsweise prinzipiell aus der EP 3 132 765 A1 bekannt, mit unterschiedlichen Polen einer Spannungsquelle verbunden sein und somit zwischen ihren Schenkeln Koagulationsspalte 20, 21 festlegen. Zwischen diesen sind ein vorzugsweise elastisches Widerlager 22 und in der gegenüberliegenden Branche 19 eine Schneidelektrode 23 angeordnet. Diese weist einen Basis- oder Befestigungsabschnitt 24 und einen Wandabschnitt 25 auf, die beide aus einem Kunststoff, vorzugsweise einem elastischen Kunststoff, bestehen, wobei in den Wandabschnitt 25 und gegebenenfalls zumindest teilweise auch in den Basisabschnitt 24 hinein ragend eine vorzugsweise aus Metall bestehende Elektrode 26 eingebettet ist. Von dieser liegt an der Stirnseite 27 lediglich eine schmale Kante 28 frei. Im Übrigen ist die Elektrode 26 so in den Wandabschnitt 25 und dem Basisabschnitt 24 eingebettet, dass sie elektrisch isoliert ist.

Das Werkzeug 17 kann auch Teil eines anderen Instruments, z.B. eines endoskopisch zu benutzenden Instruments oder eines für den offenchirurgischen Einsatz gestalteten Instruments sein.

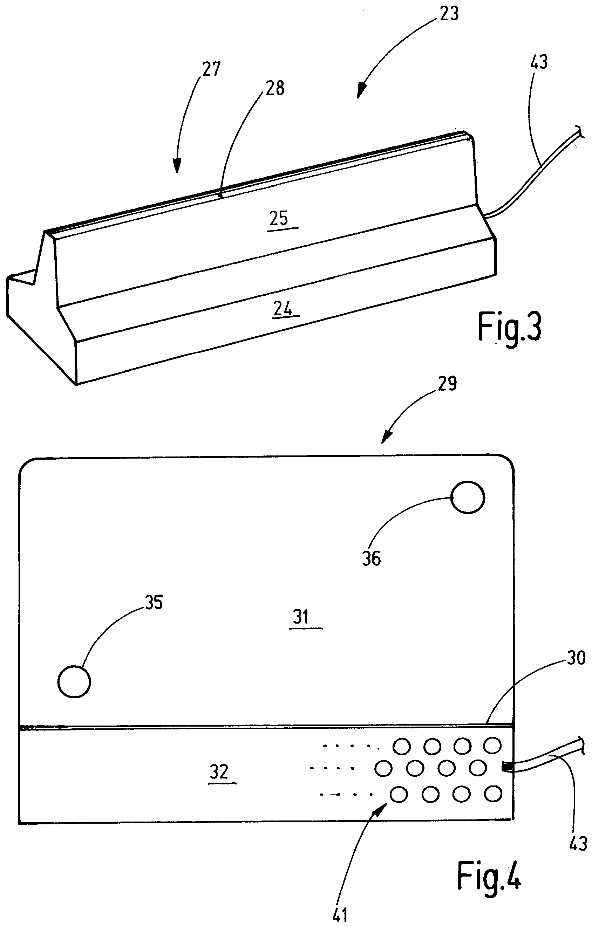

Die Herstellung der Schneidelektrode 24 geht wie folgt:
Es wird zur Herstellung von einem Materialzuschnitt 29 ausgegangen, wie er in Figur 4 veranschaulicht ist. Dieser Materialzuschnitt 29 besteht aus einem geeigneten Elektrodenmaterial, beispielsweise einer Stahllegierung, einer Titanlegierung oder aus einer elektrisch leitfähigen Keramik. Vorzugsweise besteht der Materialzuschnitt 29 aus einem federnden Material, das jedoch keine wesentliche plastische Verformbarkeit hat. Insbesondere wird ein Material bevorzugst dessen Bruchgrenze ohne vorherige plastische Verformung erreicht wird.

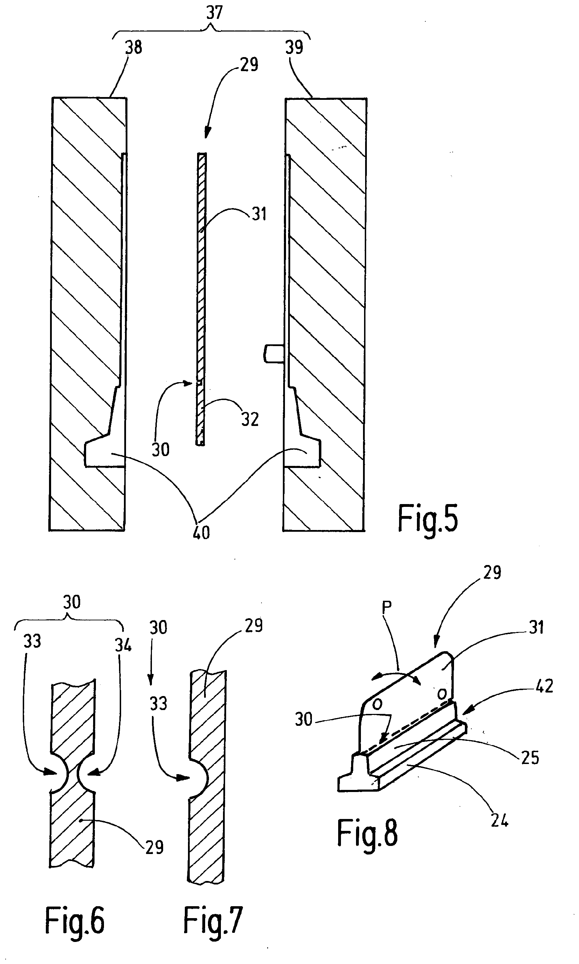

Der Materialzuschnitt 29 ist mit einer vorzugsweise linienförmigen Sollbruchstelle 30 versehen, die einen ersten Abschnitt 31 des Materialzuschnitts 29 von einem zweiten Abschnitt 32 trennt. Der zweite Abschnitt 32 bildet die spätere Elektrode 26, während der erste Abschnitt 31 nur der zeitweiligen Handhabung dient. Die Sollbruchstelle 30 ist eine linienhafte Schwächung des Materials des Materialzuschnitts 29, beispielsweise durch eine in den Materialzuschnitt 29 eingearbeitete Nut 33, wie sie aus Figur 7 ersichtlich ist. Anstelle einer lediglich in eine Flachseite des Materialzuschnitts 29 eingebrachte Nut 33 kann die Sollbruchstelle 30 auch, wie es Figur 6 zeigt, durch zwei Nute 33, 34 gebildet sein, die parallel zueinander in voneinander wegweisenden Flachseiten des Materialzuschnitts 29 angebracht sind. Die beiden Nute 33, 34 verlaufen dabei entlang gleicher Wege und sind in gleicher Position angeordnet.

Unabhängig davon, ob lediglich eine Nut 33 oder zwei Nuten 33, 34 vorgesehen sind, können diese in jedem geeigneten Verfahren, beispielsweise durch Schleifen, Fräsen, Prägen, Rollieren oder Ätzen, hergestellt werden. Der Querschnitt der Nuten 33, 34 kann dabei je nach gewähltem Verfahren und gewünschter Kantenform rund, eckig, dreieckig, viereckig, trapezförmig oder auch insbesondere im Ätzverfahren in unbestimmter Form erzeugt werden. Jedoch sind Tiefe und Breite der Nut auch beim Ätzverfahren definiert.

In dem ersten Abschnitt 31 können ein oder mehrere Positionierstrukturen, z.B. in Gestalt von Positionierlöchern 35, 36 vorgesehen sein. Diese können zum Beispiel der Positionierung des Materialzuschnitts 29 in einer Gießform 37 mit zwei Formhälften 38, 39 dienen, wie sie aus Figur 5 hervorgehen.

Die Formhälften 38, 39 sind jeweils mit einer Gravur versehen, die im Bereich des ersten Abschnitts 31 des Materialzuschnitts von ihrer Form und Tiefe so bemessen ist, dass der Materialzuschnitt 29 bei geschlossener Form 37 an dem ersten Abschnitt 31 festgehalten ist, wobei die beiden Formhälften 38, 39 flächenhaft an dem ersten Abschnitt 31 anliegen. Insbesondere schließt dabei der den ersten Abschnitt 31 haltende Bereich der Formhälften 38, 39 im Wesentlichen bündig an der Sollbruchstelle 30 ab. Bei geschlossener Form 37 entsteht dadurch um den zweiten Abschnitt 32 herum ein Hohlraum 40, in den der zweite Abschnitt 32 freitragend hinein ragt. Dieser Hohlraum 40 schließt wiederum an der Sollbruchstelle 30 bündig ab.

Zur Herstellung der Schneidelektrode 22 wird der Abschnitt 32 bei geschlossener Form 37 mit einem geeigneten Kunststoff, beispielsweise einem Silikon gefüllt, der dabei auch die Löcher 41 durchdringen kann, die in dem zweiten Abschnitt 32 zur formschlüssigen Verankerung des Kunststoffs an dem Materialzuschnitt ausgebildet sein können. Die Form der Löcher kann rund oder eckig sein.

Nach dem verfestigen des Kunststoffs und dem Öffnen der Gießform 37 kann der Materialzuschnitt 29 mit dem daran ausgebildeten Kunststoffkörper 42 aus der Gießform 37 entnommen werden. Wie in Figur 8 veranschaulicht ist, liegt die Sollbruchstelle 30 nun unmittelbar an dem oberen Ende des Wandabschnitts 25. Aus dem Kunststoffkörper 42 ragt gegebenenfalls ein an dem Abschnitt 32 des Materialzuschnitts 29 angebrachtes Kabel 43.

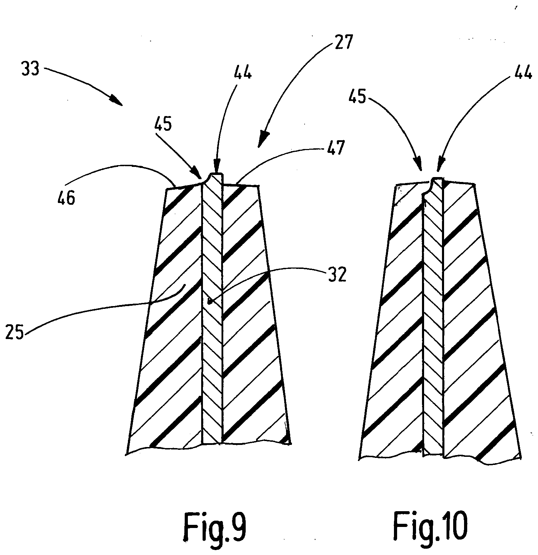

Zur Fertigstellung der Schneidelektrode 23 wird nun der erste Abschnitt 31 gegen den Kunststoffkörper 40 in der in Figur 8 durch einen Pfeil P bezeichneten Richtung schwenkend hin und her bewegt, wodurch der erste Abschnitt 31 an der Sollbruchstelle 30 von dem zweiten Abschnitt abbricht. Es bildet sich dabei wie Figur 9 zeigt, eine Bruchkante 44. Aus der ehemaligen Nut 32 ist ein Verjüngungsbereich 45 entstanden, sodass die Bruchkante 44 eine geringere Breite aufweist als die Dicke des in den Kunststoffkörper eingebetteten zweiten Abschnitts 32. Sowohl die Breite der Bruchkante 44 als auch die Dicke des zweiten Abschnitts 32 sind in Figur 9 horizontal, d.h. in jedem Fall senkrecht zur Flachseite des zweiten Abschnitts 32 zu messen.

Der Wandabschnitt 25 weist an seiner Stirnseite 27 zu beiden Seite der Bruchkante 44 ebene oder gerundete Flächenabschnitte 46, 47 auf, deren Breite vorzugsweise jeweils höchstens 10 mal so groß, weiter vorzugsweise höchstens dreimal so groß oder auch höchstens doppelt so groß ist wie die Dicke des zweiten Abschnitts 32.

Während der Wandabschnitt 25 stirnseitig unmittelbar an den Verjüngungsbereich 45 anschließen kann, wie es in Figur 9 veranschaulicht ist, kann er sich gemäß Figur 10 auch bis in den Verjüngungsbereich 45 hinein erstrecken, sodass auch dieser teilweise oder ganz von Kunststoff bedeckt ist und ausschließlich die Bruchkante 44 freiliegt. Hiermit wird eine besonders hohe Stromkonzentration erreicht.

Die Bruchkante 44 ist bruchrau und kann nicht nachgearbeitet sein. Nach dem Abbrechen des ersten Abschnitts 31 von dem zweiten umspritzten Abschnitt 32 gemäß Figur 8 ist die Schneidelektrode 23 fertig und kann in das Instrument nach Figur 1 eingebaut werden. An der Bruchkante entstehen beim Schnitt aufgrund der Rauheit lokale Stromkonzentrationen, die den Schnitt fördern. Es ist aber auch möglich, die Bruchkante nachzubearbeiten, beispielsweise durch Polieren, insbesondere Elektropolieren.

Die im Zusammenhang mit den Figuren 5 sowie 8 bis 10 beschriebenen Details gelten gleichermaßen für Materialzuschnitte 29 nach Figur 6 und Materialzuschnitte 29 nach Figur 7.

Das erfindungsgemäße Verfahren dient der Herstellung einer Schneidelektrode für ein chirurgisches Instrument 11, wobei das Verfahren einen zum Durchtrennen des Querschnitts der Schneidelektrode 23 nötigen Krafteintrag nach Anbringen des Kunststoffkörpers an der Schneidelektrode 23 vermeidet. Zur Herstellung der Schneidelektrode 23 wird von einem Materialzuschnitt 29 ausgegangen, der mit einer Sollbruchstelle 30 versehen wird. Diese wird so bemessen, dass eine Durchtrennung derselben mit geringen Kräften möglich ist, die insbesondere so gering sind, dass sie auch von dem Kunststoffkörper übertragbar sind. Die Sollbruchstelle 30 unterteilt den Materialzuschnitt 29 in einen ersten Abschnitt 31, der lediglich der Handhabung und der Positionierung des Materialzuschnitts 29 in einer Gießform 37 dient sowie in einen zweiten Abschnitt 32, der frei in einen Formhohlraum 40 ragt und von Kunststoff umspritzt wird.

Nach dem Ausformen kann der erste Abschnitt 31 leicht von der Schneidelektrode 23 abgebrochen werden. Die entstehende Bruchkante 44 bildet eine ideale Schneidkante.

### Bezugszeichen:

- 11: Instrument
- 12: Gehäuse
- 13: Griff
- 14: Handhebel
- 15: Schaft
- 16: distales Ende des Schafts 15
- 17: Werkzeug
- 18, 19: Branchen
- 20, 21: Koagulationsspalte
- 22: Widerlager
- 23: Schneidelektrode
- 24: Basisabschnitt
- 25: Wandabschnitt
- 26: Elektrode
- 27: Stirnseite
- 28: Schneidkande
- 29: Materialzuschnitt
- 30: Sollbruchstelle
- 31: erster Abschnitt
- 32: zweiter Abschnitt
- 33, 34: Nute
- 35, 36: Positionierlöcher
- 37: Gießform
- 38, 39: Formhälften
- 40: Hohlraum
- 41: Löcher
- 42: Kunststoffkörper
- 43: Kabel
- P: Pfeil
- 44: Bruchkante
- 45: Verjüngungsbereich
- 46, 47: Flächenabschnitte

## Patentansprüche

1. Verfahren zur Herstellung einer Schneidelektrode (23) für ein chirurgisches Instrument (11), insbesondere ein Fusions- und Dissektionsinstrument, mit folgenden Schritten:
Bereitstellen eines flachen Materialzuschnitts (29) aus einem elektrisch leitfähigen Material,
Anbringen einer Sollbruchstelle (30), die den Materialzuschnitt (29) in einen ersten und einen zweiten Abschnitt (31, 32) unterteilend angeordnet ist,
Einlegen des mit der Sollbruchstelle (30) versehenen Materialzuschnitts (29) in eine Gießform (37), die eine Gravur aufweist, die den ersten Abschnitt (31) anliegend fasst und die um den zweiten Abschnitt herum einen Hohlraum (40) definiert,
Einfüllen eines Kunststoffs in die Gießform (37) zur Erzeugung eines Kunststoffkörpers (42), der den zweiten Abschnitt (32) umschließt,
Öffnen der Gießform (37) und Entnehmen des Kunststoffkörpers (42) mit dem darin gefassten Materialzuschnitt (29),
Abbrechen des ersten Abschnitts (31) von dem zweiten Abschnitt (32) entlang der Sollbruchstelle (30).

2. Verfahren nach Anspruch 1, **dadurch gekennzeichnet, dass** der erste Abschnitt (31) mindestens so groß ist, wie der zweite Abschnitt (32).

3. Verfahren nach Anspruch einem der vorstehenden Ansprüche, **dadurch gekennzeichnet, dass** der erste Abschnitt (31) mittels mindestens einer Positionierstruktur (35) in der Gießform (37) lagerichtig positioniert wird.

4. Verfahren nach einem der vorstehenden Ansprüche, **dadurch gekennzeichnet, dass** der Materialzuschnitt (29) vor dem Einlegen in die Gießform (37) mit einer elektrischen Anschlussleitung (43) versehen wird.

5. Verfahren nach einem der vorstehenden Ansprüche, **dadurch gekennzeichnet, dass** die Sollbruchstelle (30) als ein linienhafter Bereich verminderter Materialstärke ausgebildet wird.

6. Verfahren nach Anspruch 5, **dadurch gekennzeichnet, dass** der Bereich verminderter Materialstärke durch mindestens eine Nut (33, 34) gebildet ist, die an mindestens einer Flachseite des Materialzuschnitts (29) angeordnet ist.

7. Verfahren nach Anspruch 6, **dadurch gekennzeichnet, dass** die Nut (33, 34) durch ein Ätzverfahren erzeugt ist.

8. Schneidelektrode für ein chirurgisches Instrument, insbesondere Schneidelektrode hergestellt nach einem Verfahren gemäß einem der vorstehenden Ansprüche, **dadurch gekennzeichnet, dass** sie einen Kunststoffkörper (42) aufweist, in den eine Elektrode (26) eingebettet ist, die eine freiliegende Bruchkante (44) aufweist, die bruchrau ist.

9. Schneidelektrode nach Anspruch 8, **dadurch gekennzeichnet, dass** der Kunststoffkörper (42) aus einem flexiblen Kunststoff, insbesondere einem elastischen Kunststoff, insbesondere Silikon ausgebildet ist.

10. Schneidelektrode nach einem der Ansprüche 8 oder 9, **dadurch gekennzeichnet, dass** die Elektrode (26) eine einheitliche konstante Dicke aufweist und in unmittelbarer Nachbarschaft der Bruchkante (44) eine reduzierte Dicke aufweist, so dass an die Bruchkante (44) anschließend ein Verjüngungsbereich (45) ausgebildet ist.

11. Schneidelektrode nach Anspruch 10, **dadurch gekennzeichnet, dass** der Kunststoffkörper (42) bis an den Verjüngungsbereich (45) heranreichend ausgebildet ist.

12. Schneidelektrode nach Anspruch 10, **dadurch gekennzeichnet, dass** der Kunststoffkörper (42) den Verjüngungsbereich (45) übergreifend ausgebildet ist.

13. Schneidelektrode nach einem der Ansprüche 8 bis 12, **dadurch gekennzeichnet, dass** die Schneidelektrode (23) von dem Kunststoff durchsetzte Löcher (41) aufweist.

14. Schneidelektrode nach einem der Ansprüche 8 bis 13, **dadurch gekennzeichnet, dass** die Breite der Bruchkante (44) geringer ist als die Dicke der Elektrode (26), vorzugsweise höchstens halb so groß ist, wie die Dicke der Elektrode (26).

15. Chirurgisches Instrument mit einer Schneidelektrode (23) nach einem der Ansprüche 8 bis 14.

## Claims

1. Method of manufacturing a cutting electrode (23) for a surgical instrument (11), in particular a fusion and dissection instrument, with the following steps:
provision of a flat material blank (29) from an electrically conductive material, application of a nominal break point (30) which is arranged so as to divide the material blank (29) into a first and a second portion (31, 32),
laying of the material blank (29) provided with the nominal break point (30) in a casting mould (37) which has an engraving and holds the first portion (31) with contact and defines a cavity (40) around the second portion,
filling of a plastic into the casting mould (37) in order to produce a plastic body (42) surrounding the second portion (32),
opening of the casting mould (37) and removal of the plastic body (42) with material blank (29) held therein,
breaking of the first portion (31) away from the second portion (32) along the nominal break point (30).

2. Method according to claim 1, **characterised in that** the first portion (31) is at least as large as the second portion (32).

3. Method according to any of the preceding claims, **characterised in that** the first portion (31) is positioned in the correct position in the casting mould (37) by means of at least one positioning structure (35).

4. Method according to any of the preceding claims, **characterised in that** the material blank (29) is provided with an electrical connecting line (43) before laying in the casting mould (37).

5. Method according to any of the preceding claims, **characterised in that** the nominal break point (30) is configured as a linear region of reduced material thickness.

6. Method according to claim 5, **characterised in that** the region of reduced material thickness is formed by at least one groove (33, 34) which is arranged on at least one flat side of the material blank (29).

7. Method according to claim 6, **characterised in that** the groove (33, 34) is produced by an etching process.

8. Cutting electrode for a surgical instrument, in particular a cutting electrode produced using a method according to any of the preceding claims, **characterised in that** it has a plastic body (42) in which an electrode (26) is embedded which has an exposed, rough-broken break edge (44).

9. Cutting electrode according to claim 8, **characterised in that** the plastic body (42) is made from a flexible plastic, in particular an elastic plastic, in particular silicone.

10. Cutting electrode according to one of claims 8 and 9, **characterised in that** the electrode (26) has a uniform constant thickness and a reduced thickness in the direct vicinity of the break edge (44) so that a tapering region (45) is formed adjoining the break edge (44).

11. Cutting electrode according to claim 10, **characterised in that** the plastic body (42) is configured so as to extend up to the tapering region (45).

12. Cutting electrode according to claim 10, **characterised in that** the plastic body (42) is configured so as to extend over the tapering region (45).

13. Cutting electrode according to any of claims 8 to 12, **characterised in that** the cutting electrode (23) has holes (41) through which the plastic passes.

14. Cutting electrode according to any of claims 8 to 13, **characterised in that** the width of the break edge (44) is less than the thickness of the electrode (26), preferably at most half as great as the thickness of the electrode (26).

15. Surgical instrument with a cutting electrode (23) according to any of claims 8 to 14.

## Revendications

1. Procédé de fabrication d'une électrode de découpage (23) pour un instrument chirurgical (11), en particulier un instrument de fusion et de dissection, avec des étapes suivantes :
de fourniture d'une découpe de matériau (29) plate issue d'un matériau électriquement conducteur,
de mise en place d'un point de rupture théorique (30), qui est disposé de manière à diviser la découpe de matériau (29) en une première et en une deuxième section (31, 32),
de placement de la découpe de matériau (29) pourvue du point de rupture théorique (30) dans un moule de coulée (37), qui présente une gravure, qui contient en appui la première section (31) et qui définit un espace creux (40) tout autour de la deuxième section,
de transvasement d'une matière synthétique dans le moule de coulée (37) pour produire un corps en matière synthétique (42), qui renferme la deuxième section (32), d'ouverture du moule de coulée (37) et de retrait du corps en matière synthétique (42) avec la découpe de matériau (29) contenue dans celui-ci,
de détachement par rupture de la première section (31) de la deuxième section (32) le long du point de rupture théorique (30).

2. Procédé selon la revendication 1, **caractérisé en ce que** la première section (31) est au moins aussi grande que la deuxième section (32).

3. Procédé selon l'une quelconque des revendications précédentes, **caractérisé en ce que** la première section (31) est positionnée en position correcte dans le moule de coulée (37) au moyen d'au moins une structure de positionnement (35).

4. Procédé selon l'une quelconque des revendications précédentes, **caractérisé en ce que** la découpe de matériau (29) est pourvue d'un câble de raccordement (43) électrique avant le placement dans le moule de coulée (37) .

5. Procédé selon l'une quelconque des revendications précédentes, **caractérisé en ce que** le point de rupture théorique (30) est réalisé en tant qu'une épaisseur de matériau réduisant une zone linéaire.

6. Procédé selon la revendication 5, **caractérisé en ce que** la zone à épaisseur de matériau réduite est formée par au moins une rainure (33, 34), qui est disposée sur au moins un côté plat de la découpe de matériau (29).

7. Procédé selon la revendication 6, **caractérisé en ce que** la rainure (33, 34) est produite par un procédé de gravure.

8. Electrode de découpage pour un instrument chirurgical, en particulier électrode de découpage fabriquée selon un procédé selon l'une quelconque des revendications précédentes, **caractérisée en ce qu'**elle présente un corps en matière synthétique (42), dans lequel est intégrée une électrode (26) qui présente une arête de rupture (44) à découvert, qui est rugueuse.

9. Electrode de découpage selon la revendication 8, **caractérisée en ce que** le corps en matière synthétique (42) est réalisé à partir d'une matière synthétique flexible, en particulier d'une matière synthétique élastique, en particulier de silicone.

10. Electrode de découpage selon l'une quelconque des revendications 8 ou 9, **caractérisée en ce que** l'électrode (26) présente une épaisseur constante uniforme et présente, à proximité immédiate de l'arête de rupture (44), une épaisseur réduite de telle sorte qu'une zone de rétrécissement (45) est réalisée dans le prolongement de l'arête de rupture (44).

11. Electrode de découpage selon la revendication 10, **caractérisée en ce que** le corps en matière synthétique (42) est réalisé de manière à atteindre la zone de rétrécissement (45).

12. Electrode de découpage selon la revendication 10, **caractérisée en ce que** le corps en matière synthétique (42) est réalisé de manière à surmonter la zone de rétrécissement (45).

13. Electrode de découpage selon l'une quelconque des revendications 8 à 12, **caractérisée en ce que** l'électrode de découpage (23) présente des trous (41) traversés par la matière synthétique.

14. Electrode de découpage selon l'une quelconque des revendications 8 à 13, **caractérisée en ce que** la largeur de l'arête de rupture (44) est inférieure à l'épaisseur de l'électrode (26), de préférence est égale au maximum à la moitié de l'épaisseur de l'électrode (26).

15. Instrument chirurgical, avec une électrode de découpage (23) selon l'une quelconque des revendications 8 à 14.
